# EUROPÄISCHE PATENTSCHRIFT

(11) **EP 1 449 559 B1**
(45) Veröffentlichungstag und Bekanntmachung des Hinweises auf die Patenterteilung: **11.07.2007**
(21) Anmeldenummer: 04002347.5
(22) Anmeldetag: 03.02.2004
(51) Int. Cl.: A61M 16/12, A61M 16/00

(54) **Vorrichtung zur dosierten Abgabe eines therapeutischen Gases**
Device for metered delivery of therapeutic gas
Dispositif pour distribution dosée de gaz thérapeutique

(30) Priorität: 18.02.2003 DE 10306766
(43) Veröffentlichungstag der Anmeldung: 25.08.2004
(73) Patentinhaber: INO Therapeutics GmbH, 2345 Brunn am Gebirge (AT)
(72) Erfinder: Krebs, Christian, 2331 Vösendorf (AT)
(74) Vertreter: Kasseckert, Rainer

(56) Entgegenhaltungen:
- EP-A- 0 861 672
- US-A- 3 721 239
- US-A- 4 265 239
- US-A- 4 617 924
- US-A- 5 507 280
- US-A- 6 131 571
- US-A- 6 142 147

## Beschreibung

Die Erfindung betrifft eine Vorrichtung zur dosierten Abgabe eines therapeutisch wirksamen Gases an einen Patienten mit einem Spülschritt zum Entfernen schädlicher Substanzen aus Teilen des gasführenden Systems.

Stickstoffmonoxid, NO, ist mittlerweile als Medikament gegen pulmonale Hypertension bekannt, also als Vasodilator. Der Vorteil der Behandlung mit den vielfach beschriebenen NO-Gemischen ist, dass NO nur lokal, also im pulmonalen Kreislauf, und nicht systemisch wirksam ist. Das NO-Gasgemisch wird dem Patienten per Inhalationen appliziert. Das bedeutet, dass dem therapeutisch wirksamen Gas weitere Gasbestandteile, insbesondere Luft, Sauerstoff oder andere sauerstoffhaltige Gase, mittels verschiedener Techniken zugemischt werden.

Aus der EP 621 051 A2 ist es bekannt, bei künstlich beatmeten Patienten das Stickoxidgemisch volumenstromproportional - also dem inspiratorischen, eingeatmeten Fluss des Luft-Sauerstoffgemisches folgend - zuzudosieren. Der Fluss des NO-Gemisches kann auch dem eingeatmeten Volumen entsprechend zudosiert werden.

Aus der US 5,839,433 oder der WO 98/31282 sind weitere Techniken der Administration von NO zu einem Patienten bekannt. Beschrieben sind dort die sog. Spike-, Peak- oder Puls-Techniken. Hierzu wird meist ein Ventil für eine gewisse Zeit geöffnet und dann während der inspiratorischen Phase wieder geschlossen, um somit ein gewisses Volumen an NO der Lunge des Patienten zuzuführen. Diese Technik wird meist - aber nicht ausschließlich - beim spontanen Atmer angewandt, also bei dem Patienten, welcher nicht an einer Beatmungsmaschine angeschlossen ist.

Welche Methode bei der NO-Dosierung auch gewählt wird, bleibt stets ein negativer Nebeneffekt der NO-Behandlung, nämlich dass sich im Laufe der Zeit NO zu NO₂ umwandelt, wenn NO mit einem sauerstoffhaltigen Gas gemischt ist. Da Luft zu 21 % Sauerstoff enthält, erfolgt dies auch an Luft. Vergeht mehr Zeit, während NO in Luft oder Sauerstoff präsent ist, entsteht auch mehr NO₂. Beispielsweise entsteht mehr NO₂ beim Starten, beim Unterbrechen oder bei der Wiederaufnahme der NO-Therapie - da mehr Zeit für die Oxidation zur Verfügung steht. Also immer, wenn Zeit zum Weiteroxidieren zur Verfügung steht und sich in den Zuführschläuchen und in den Zwischenräumen zwischen den einzelnen Geräten oder aber im Patientenkonnektor, welcher beispielsweise eine Nasenbrille oder Maske sein kann, Luft und NO befinden. In diesen Pausen steht meist noch NO in den Leitungen und kann dann zu NO₂ reagieren. Das im System befindliche Gas enthält dann in der Regel relativ hohe NO₂-Anteile, die entweder ausgespült werden müssen oder aber zum Patienten gelangen. Da schädliche Effekte bereits ab geringsten Konzentrationen auftreten, sollten auch kleinste Quantitäten von NO₂ nicht toleriert werden. Dieses Problem tritt insbesondere - aber nicht ausschließlich - bei spontan atmenden Patienten auf, wobei beispielsweise durch schlecht sitzende Nasenbrillen oder schlecht sitzende Masken, das gewünschte Triggern - also das Zudosieren von NO Gemischen - ausbleibt. Somit steht dann das Gemisch in der Zuleitung und reagiert in Präsenz mit Sauerstoff zu NO₂. Aber auch durch eine andere Fehldetektion des Sensors, welcher den Start der Inspiration und damit den NO-Puls an den Patienten triggert, oder durch andere Ergebnisse, welche die Einleitung des oder der nächsten Gasgemischpulses verhindern, kann das in der Zuleitung befindliche NO-Gemisch mit Luft/Sauerstoff zur erhöhten NO₂-Bildung führen. Dies führt dann - nach Wiedertriggerung - dazu, dass der Patient ein Gas mit erhöhtem NO₂-Gehalt erhält.

Zur Behebung dieses Nachteils sind aus der US 6,142,147, der US 6,125,846 und der US 6,109,260 jeweils eine Vorrichtung und ein Verfahren bekannt, welche einen Ausspülprozess umfassen. Der Ausspülprozess erfolgt so, dass bei Auftreten einer längeren Applikationspause oder aber eine Apnoe-Pause das NO₂-Gemisch von einem längeren Dauervolumenstrom des Behandlungsgases, nämlich NO, durchgespült wird, um das NO₂-Gemisch auszuspülen. Nachteilig hierbei ist, dass das Spülgas selbst NO-haltig ist und daher bei der nächsten Pause wieder zu NO₂ reagieren kann. Im schlechtesten Fall atmet der Patient dann wieder NO₂ ein. Das Dokument US 6,142,147 representiert der nächstliegende Stand der Technik und zeigt alle Merkmale des Oberbegriffs des Anspruchs 1.

Aufgabe der Erfindung ist es daher, diese Verfahren dahingehend zu verbessern, dass sie sicherer werden und der Patient zuverlässiger vor dem Einatmen von NO₂ oder anderen schädlichen Gasen geschützt ist.

Diese Aufgabe wird erfindungsgemäß gelöst von einer Vorrichtung, bei der ein Schritt des Spülens mit einem anderen Gas vorgesehen ist.

Nach der Erfindung wird nicht mit dem NO-haltigen therapeutischen Gas sondern mit einem anderen Gas, insbesondere mit Luft, Sauerstoff, Stickstoff oder anderen Edelgasen oder Gemischen - all diese Gase werden zur Applikation am Patienten oder aber ausschließlich zum Spülen verwendet - das Gas mit erhöhter NO₂-Konzentration aus dem System ausgespült. Der Ausspülprozess entspricht dem des genannten Standes der Technik, die Spül-Medien sind jedoch unterschiedlich. Auch andere therapeutisch wirksame Gase können zum Spülen verwendet werden.

Das mit dem schädlichen Gas kontaminierte Gasgemisch kann in dem System mittels einer Pumpe abgesaugt werden, so dass es nicht mehr zum Patienten gelangen kann. Solche Lösung zu dem Problem gehört aber nicht zur Erfindung.

Das therapeutisch wirksame Gas ist beispielsweise NO. Der Mechanismus, nachdem es zu NO₂ reagiert, wenn es mit Sauerstoff in Berührung kommt, wurde bereits oben beschrieben. Ein anderes therapeutisch wirksames Gas ist CO. Auch dieses Gas kann sich bei Zudosieren in Leitungen sammeln und könnte dann in unerwünscht hohen Konzentration dem Patienten zugegeben werden. Bei der Therapie mit CO kann ein solcher Spülschritt mit einem anderen Gas oder ein Absaugschritt also auch vorteilhaft sein. Andere therapeutisch wirksame Gase sind: CO₂-Gemische zur Atemstimulierung, H₂ Gemische, N₂O Gemische, SF₆ Gemische, Nitrosoethanol, Anästhesiegase (wie z.B. Isoflouran und andere volatile Anästhesiegase, Xenon) um beispielsweise den Patienten von der Anästhesie abzusetzen.

Wenn also einer der beschriebenen Fälle eintritt (Sensor detektierte keine Atemzüge mehr und dosiert nicht, es beginnt oder endet eine längere Unterbrechung der NO-Applikation oder ein NO-Monitor zeigt einen erhöhte NO₂-Gehalt an), kann das System bis zum Patienten, also von der Gasflasche bis möglichst zur Nasenbrille, vollkommen von dem NO₂-erhöhten Gemisch ausgesaugt oder ausgespült werden. Auch die geräteinternen Gaselemente wie Schläuche und Ventile sollten so weit wie möglich vom NO₂ oder dem anderen schädlichen Gas befreit werden. Alle Toträume, Zuleitungen oder Ventile sollten gespült werden.

Auch beim Aussaugen sollten möglichst alle vom Gas berührten Leitungsteile (Nasenbrille, Maske, Schläuche, Ventile, Schlauchleitungen, Sensoren, Toträume) umfasst werden.

Das Spülen oder Saugen kann zeitgesteuert, sensorgesteuert oder eventgesteuert (z.B. der Event, dass das therapeutische Gas an den Patienten ausgeschaltet wird - also Dosierung Stopp) erfolgen. Zeitgesteuert meint, dass der Spül- oder Saugschritt eine bestimmte, voreingestellte Zeitdauer durchgeführt wird und/oder nach einer bestimmten Zeitsequenz. Sensorgesteuert meint, dass der Spül- oder der Saugschritt in Abhängigkeit von einer gemessenen Konzentration und/oder von einem gemessenen Durchfluss und/oder von einem gemessenen Druck erfolgt. Insbesondere beim Saugen empfiehlt es sich, bis zu einem gewünschten Restdruck auszusaugen, um sicherzugehen, dass der größte Teil der schädlichen Gas entfernt ist.

Die Ausführungsformen der Erfindung werden anhand von Figuren 1-6 und 13, von insgesamt 14 Figuren, näher erläutert.

Es zeigen:
- Figuren 1 - 6 und 13:: je eine Vorrichtung zum Durchführen einer erfindungsgemäßen Spülung, und
- Figuren 7 - 12 und 14:: je eine Vorrichtung zur Durchführung eines Saugschrittes.

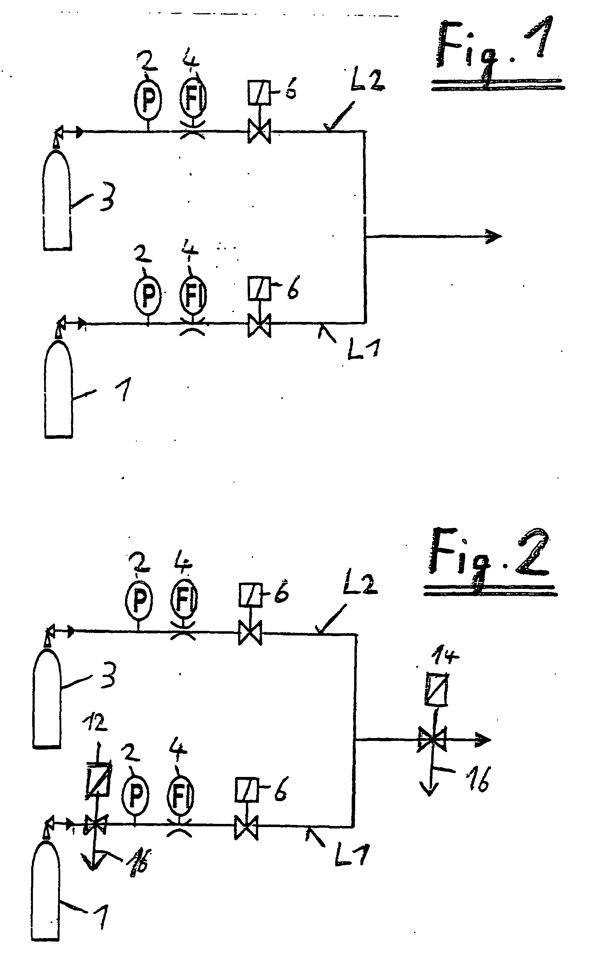

Figur 1 zeigt mit L1 eine herkömmliche erste Gasleitung, die von einer ersten Gasflasche 1, die ein Therapiegas, zum Beispiel NO (meist gelöst in Stickstoff), enthält, zu einem Patienten (Pfeil) führt. Die Leitung L1 enthält ein Druckmessgerät 2, einen Durchflussmesser 4 und ein Ventil 6 zum Regeln und Steuern des Durchflusses. L1 kann auch ohne Druckmesseinrichtung oder Durchflussmesser, oder mit den Geräten an anderen Stellen - nämlich beispielsweise nach dem Steuer- oder Regelventil - ausgeführt sein. Erfindungsgemäß ist nun eine zweite Gasleitung L2 vorgesehen, mittels derer ein anderes Gas, nämlich das Spülgas,. hier im Beispiel Sauerstoff aus der Spülgasflasche 3, durch die Leitungen bis zum Patienten gespült werden kann. Auch diese Leitung L2 enthält wieder ein Druckmessgerät 2, ein Durchflussregler 4 und ein Ventil 6, diesmal für den Sauerstoff. Die Zusammenführung von L1 und L2 sind nur symbolisch und nicht maßstäblich zu verstehen.

Die Funktion der erfindungsgemäßen Vorrichtung ist wie folgt: Zum Behandeln des Patienten mit NO wird nur die Leitung L1 benötigt. Dort kann puls-, sensor-, Volumen-, Volumenstrom- oder zeitgesteuert NO in der gewünschten Dosis oder Quantität dem Patienten gegeben werden. Tritt dann eine Behandlungspause auf, oder ein anderer Fall, nachdem der NO₂-Gehalt in der Leitung steigt, kann ein Spülschritt durchgeführt werden, in dem das NO-Ventil 6 der Leitung L1 geschlossen und das O₂-Ventil 6 der Leitung L2 geöffnet ist. So wird O₂ bis zum Patienten gespült und damit das vorher in den Leitungen befindliche Gas, welches NO₂ oder auch NO enthielt, herausgespült.

Figur 2 zeigt eine weitere Ausführung der Erfindung, bei der gleiche Teile zur Figur 1 mit den gleichen Bezugszeichen beziffert sind. Unterschiedlich ist hier, dass nunmehr nahe der Therapiegasflasche 1 ein Absperrventil 12 vorgesehen ist, welches zusätzlich eine Leitung ins Freie 16 aufweist. Ebenso ist in Figur 2 ein zweites Absperrventil 14 vorgesehen, welches ebenfalls eine Leitung 16 aufweist, die ins Freie führt. Möglich sind Ausführung der Figur 2 entweder nur mit Ventil 12 oder nur mit Ventil 14 oder mit beiden Ventilen. Gemeinsam ist beiden Konfigurationen, dass das Ventil 12 oder das Ventil 14 jeweils eine Leitung 16 gegen die Atmosphäre öffnen können. Somit kann bis direkt zur Flasche 1 gespült werden. Ventil 12 kann z.B. zum Spülschritt so geschaltet werden, dass es gegenüber der Therapiegasflasche 1 geschlossen ist und gegenüber der Leitung 16 offen. Das Gas aus der Spülleitung L2 fließt dann durch die Leitung L1 bis zum Ventil 12 und dort ins Freie. Ist auch Ventil 14 vorgesehen, so kann von dort aus das Spülgas bis zum Patienten gehen oder durch die Leitung 16 vor dem Patienten ins Freie geleitet werden. Unerwünschte Gasgemische können so bereits vor dem Patienten ausgespült werden, es kann auch zwischen Patient und Atmosphäre umgeschaltet werden.

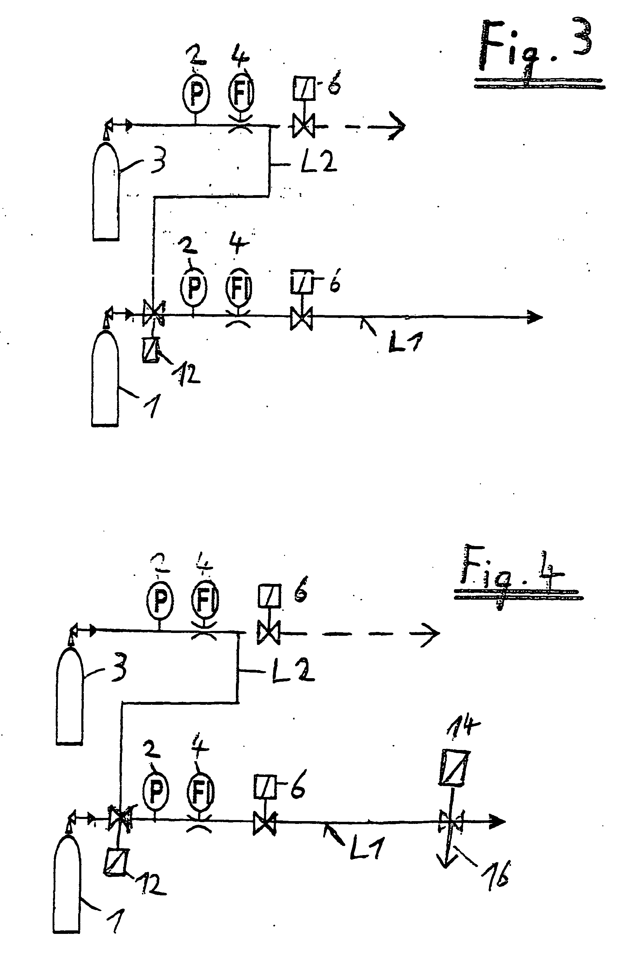

Figur 3 zeigt eine weitere Ausführung, bei der die Spülleitung L2 direkt vor der Therapiegasflasche 1 angeordnet ist, nämlich am Ventil 12. Optional, und deshalb gestichelt gezeichnet, kann eine zweite Leitung mit Ventil 6 zum Patienten (Pfeil) vorgesehen sein. Über diese Leitung kann dem Patienten ein zweites Therapiegas aus der Flasche 3 zugeführt werden. Dies kann jedes beliebige Therapiegas sein, das neben dem anderen Therapiegas eine Wirkung aufweist. Sinnvoll kann auch die Gabe eines sauerstoffenthaltenden Gases wie O₂ in der Flasche 3 oder wie Luft oder wie eines anderen sauerstoffhaltigen Gemisches sein. Dieses Gas kann dann zum Beatmen und in einem zweiten Schritt zum Spülen verwendet werden. Zum Behandeln des Patienten mit dem ersten Therapiegas wird das Ventil 12 so geschaltet, dass das erste Therapiegas aus der Gasflasche 1 bis zum bis zum Patienten (Pfeil) geleitet wird. Zum Spülen wird das Ventil 12 so umgeschaltet, dass die Flasche 1 abgeschaltet ist. Dann beginnt die Spülung der gesamten Leitung L2 und der Leitung L1 bis zum Patienten.

Figur 4 zeigt eine weitere Ausführung, wobei hier gegenüber der Figur 3 zusätzlich noch in Nähe des Patientenanschlusses das Ventil 14 vorgesehen ist, welches eine Leitung 16 ins Freie umfasst. Mittels dieses Ventils 14 kann ein Durchspülen durch die Leitung 16, die ins Freie führt, durchgeführt werden. Optional ist wieder das Ventil 6 mit der zweiten, gestrichelten Leitung für das zweite Therapiegas aus Flasche 3 zum Patienten gezeichnet.

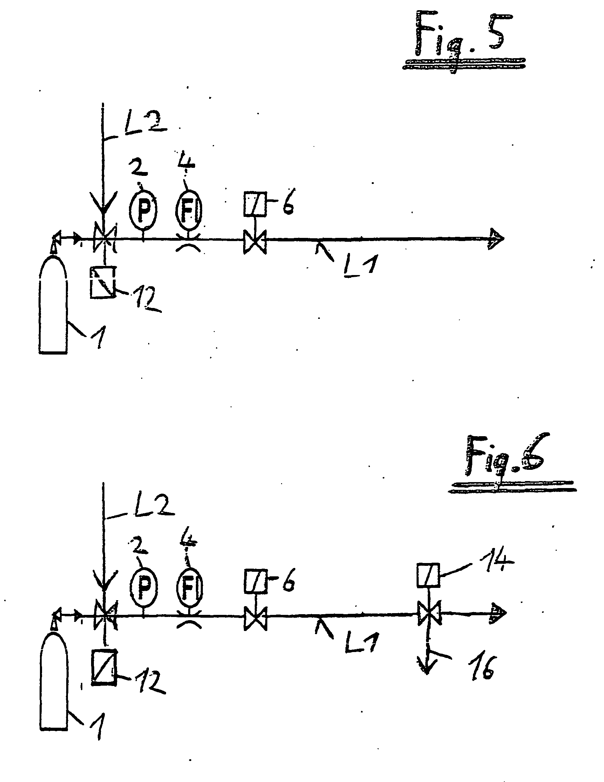

Figur 5 zeigt eine weitere Ausführung, wobei hier keine Druckgasquelle für das andere Gas verwendet wird, sondern der haus- oder klinikeigene Gasanschluss, insbesondere für Druckluft, verwendet wird. Die Leitung L2 besteht also aus einem Anschluss an das Druckluftsystem der Klinik. Zum therapeutischen Behandeln des Patienten ist das Ventil 12 auf Durchgang zwischen Gasflasche 1 und Ventil 6 geschaltet. Zum Spülen ist das Ventil 12 so geschaltet, dass der Weg zur Gasflasche 1 verschlossen ist. Druckluft aus der Leitung L2 kann dann durch das ganze System fließen und die Leitung L1 bis zum Patienten reinigen.

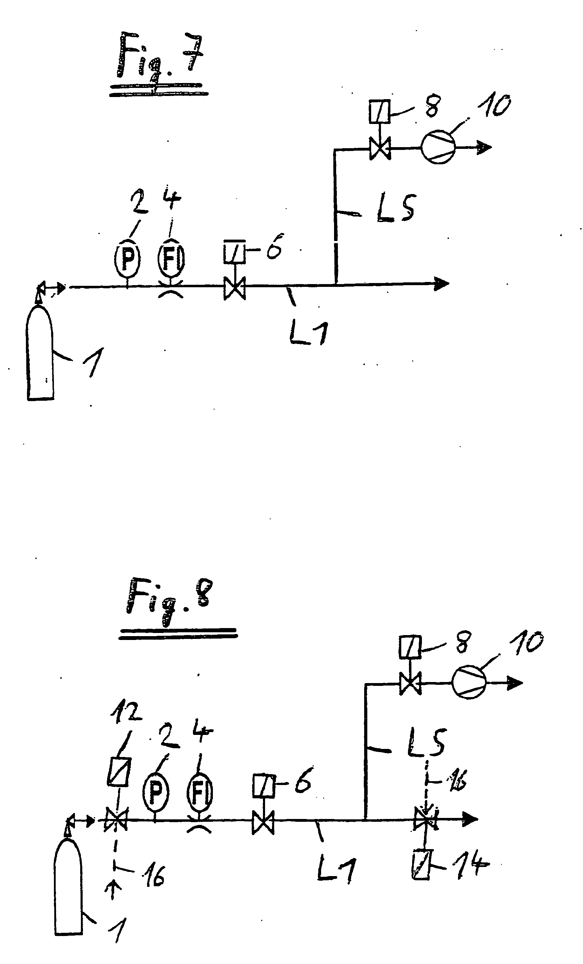

Figur 6 zeigt eine weitere Ausführung, die der Ausführung der Figur 5 ähnlich ist. Unterschiedlich ist hier ein Ventil 14 in der Nähe des Patienten, über welches eine Leitung 16, die ins Freie führt, freigeschaltet werden kann. Die Druckluft aus der Leitung L2 kann dann durch das gesamte System bis zum Ventil 14 kurz vor dem Patentenanschluss geleitet werden und so schädliche Gasbestandteile aus der Leitung L1 entfernen.

Figur 7 zeigt eine andere Lösung zu dem Problem. Gleich zur Figur 1 ist der Teil der ersten Gasleitung L1, die von der Gasvorratsflasche 1 mit dem Therapiegas zum Patienten (unterer Pfeil) führt. Neu ist nun, dass eine Saugleitung LS vorgesehen ist, die ein Saugleitungsventil 8 und ein saugendes Element 10 umfasst. Dies kann beispielsweise eine Vakuumpumpe oder aber auch der Vakuumanschluss im Krankenhaus sein. Soll nach einer Behandlungspause oder vor einer Neubehandlung der Patient wieder mit dem Therapiegas behandelt werden, kann nun erfindungsgemäß die Leitung L1 dadurch von Restgasbeständen befreit werden, dass das Ventil 8 geöffnet und die Vakuumpumpe 10 in Betrieb gesetzt wird. Die Vakuumpumpe 10 saugt dann die kontaminierten Teile des Systems leer. Nach Schließen des Saugleitungsventils 8 kann der normale Behandlungsmodus durch Öffnen des Ventils 6 erfolgen.

Figur 8 zeigt eine weitere Lösung bei der ein Ventil 12 in der Nähe der Gasflasche 1 angeordnet ist und ein Ventil 14 in der Nähe des Patienten. Wiederum kann entweder nur das Ventil 12 eingesetzt werden oder nur das Ventil 14 oder beide Ventile. Bei geschlossenen Ventilen 12 und 14 kann L1 leergesaugt werden. Optional, daher gestrichelt gezeichnet, können auch eine oder zwei Leitungen 16 aus dem Freien zu den Ventilen 12 und 16 führen. Beim Saugen über die Vakuumpumpe 10 kann dann beim Öffnen des Ventils 12 ein Luftstrom aus der Umgebung angesaugt werden, der z.B. durch das Ventil 12 oder das Ventil 14 in den Gaskreislauf gelangen kann. Die Leitung L1 wird somit nicht nur leergesaugt sondem auch mit Umgebungsluft gespült. Bei Schließen eines der beiden Ventile 12 oder 14 wird nur durch die jeweils andere Leitung 16 Luft von außen oder vom Patientengerät her eingesaugt. Beim Schließen beider Ventile wird die Leitung L1 leergesaugt.

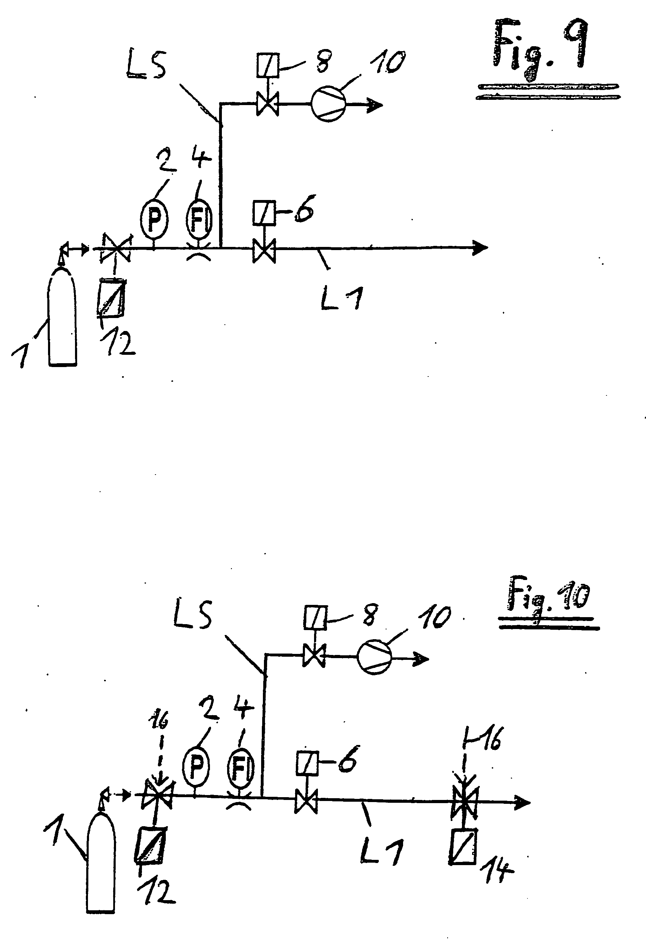

Figur 9 zeigt eine weitere Lösung, wobei die Saugleitung LS hier an einem anderen Ort angeordnet ist. Sie befindet sich nicht patientenseitig vom Ventil 6, sondern auf der anderen Seite. Zusätzlich ist hier das Ventil 12 vorgesehen, welches ein Absperren des Gaskreislaufes L1 direkt vor der Therapiegasflasche 1 erlaubt und so ein Leersaugen eines noch größeren Bereiches der Leitung L1 ermöglicht.

In Figur 10 ist zusätzlich zur Figur 9 noch das Ventil 14 patientenseitig vorgesehen, wobei hier eine Saugleitung 16 optional (daher gestrichelt gezeichnet) vorgesehen sein kann. Dann kann beim Saugen wieder Luft durch die Saugleitung 16 und das Ventil 14 in die Leitung L1 gelangen. Ebenso kann beim Ventil 12 optional eine Saugleitung 16 vorgesehen sein, durch die auch dieser Teil des Systems belüftet werden kann.

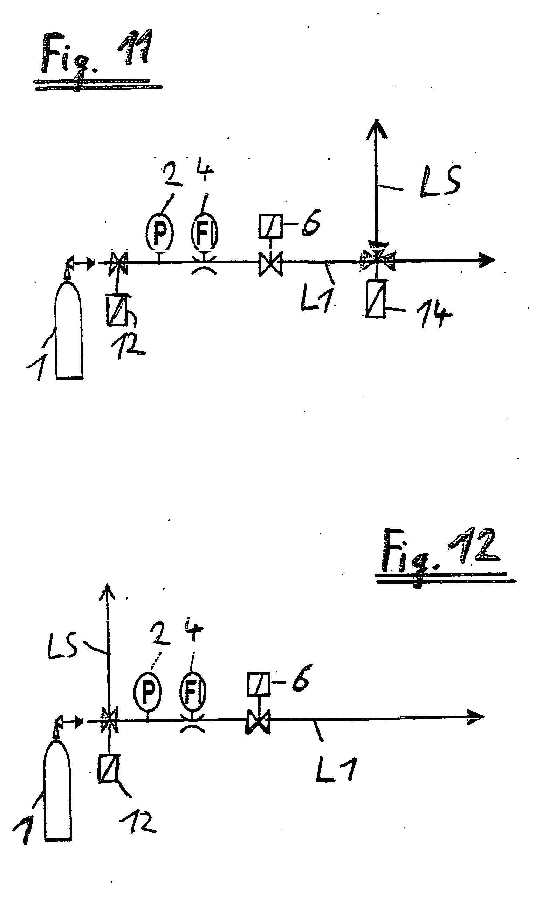

Figur 11 zeigt eine weitere Lösung des Problems, wobei die Saugleitung LS über ein 3/2-Wegeventil 14 an L1 angeschlossen ist. Ein weiteres Ventil 12 dient zum Abtrennen der Gasflasche 1 und erlaubt somit das Ausspülen der kompletten Leitung L1. Eine eigene Saugeinrichtung oder Vakuumpumpe ist hier nicht vorgesehen, statt dessen wird die Saugleitung LS an das krankenhauseigene Leitungsnetz für Unterdruck angeschlossen.

Figur 12 zeigt eine ähnliche Lösung, nur liegt der Saugleitungsanschluss LS jetzt direkt in der Nähe der Gasflasche 1 beim Absperrventil 12. Auch auf diese Weise kann die Leitung L1 fast komplett von störenden Gasbestandteilen gereinigt werden.

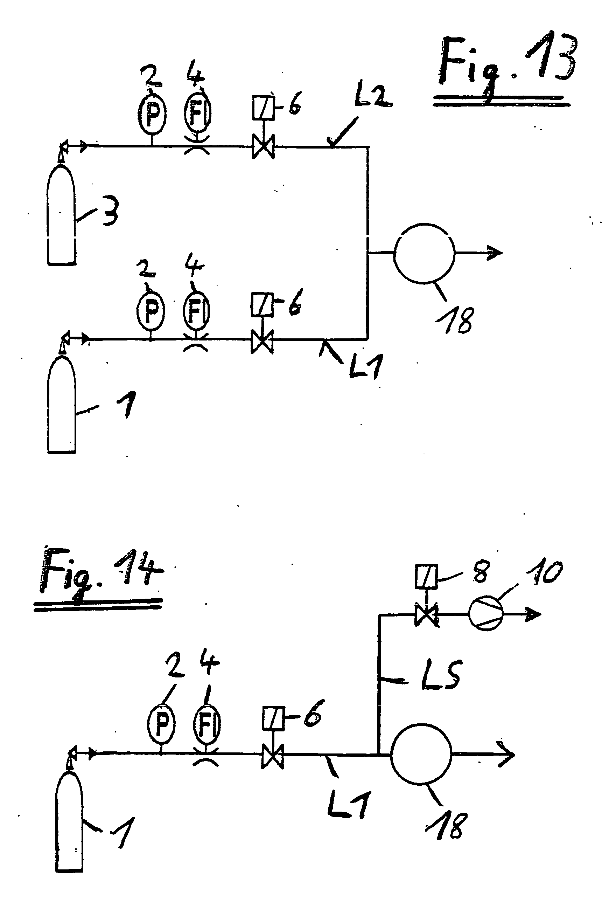

Figur 13 entspricht im Wesentlichen der Figur 1. Allerdings ist in Figur 13 zusätzlich ein Aerosolvernebler 18 vorgesehen, der eine oder mehrere therapeutisch wirksame Substanzen zur Leitung L1 zugeben kann. Auch dieser Vernebler kann vom Spülgas aus der Gasflasche 3 gespült werden.

Ein solcher Aerosolvernebler 18 kann beispielsweise auch in den Ausführungen der Figuren 2, 5 oder 6 eingesetzt sein. Er befindet sich dann bevorzugt in Figur 2 vor dem Ventil 16, womit "vor" meint, dass der Aerosolvernebler 18 sich in Strömungsrichtung vor dem Ventil 16 befindet. In Figur 5 ist er bevorzugt vor dem Patienten angeordnet, in Figur 6 bevorzugt zwischen den Ventilen 6 und 14.

Die Lösung auf der Figur 14 entspricht im Wesentlichen der Lösung auf der Figur 7. Allerdings ist dort zusätzlich ein Aerosolvernebler 18 vorgesehen, der eine oder mehrere therapeutisch wirksame Substanzen zur Leitung L1 zugeben kann. Auch dieser Vemebler 18 kann über die Leitung LS leergesaugt werden.

Ein solcher Aerosolvernebler 18 kann beispielsweise auch in den Lösungen auf den Figuren 8,9 und 10 eingesetzt sein. In Figur 8 befände er sich bevorzugt vor dem Ventil 14, in Figur 9 bevorzugt vor dem Patienten und in Figur 10 bevorzugt wieder vor dem Ventil 14.

## Patentansprüche

1. Vorrichtung zur dosierten Abgabe eines oder mehrerer therapeutisch wirksamer Gase aus der Gruppe NO, CO, CO₂-Gemische zur Atemstimulierung, H2-Gemische, N₂O-Gemische, SF6-Gemische, Nitrosethanol oder Anästhesiegase, mit einer ersten Leitung (L1), die dieses Gas (Therapiegas) zu einem Patienten führt, und mit einer zweiten Leitung (L2), die ein anderes Gas (Spülgas) führt, **gekennzeichnet durch** eine mit einem Absperrventil (14) versehene Leitung (16), die ins Freie führt, so dass schädliche oder unerwünschte Substanzen aus dem gasführenden System oder aus Teilen des gasführenden Systemes mit dem anderen Gas (Spülgas) **durch** die ins Freie führende Gasleitung (16) ausgespült werden können.

2. Vorrichtung nach Anspruch 1, **gekennzeichnet durch** eine zweite mit einem Absperrventil (12) versehene, ins Freie führende Leitung (16).

## Claims

1. Device for the metered administration of one or more therapeutically effective gases selected from the group consisting of NO, CO, CO₂ mixtures used to stimulate breathing, H2 mixtures, N₂O mixtures, SF6 mixtures, nitrosoethanol or anaesthetic gases, comprising a first line (L1), which supplies this gas (therapeutic gas) to a patient, and comprising a second line (L2), which carries another gas (purge gas), **characterized by** a line (16) being provided with a shut-off valve (14) and leading to the atmosphere, so that harmful or undesired substances can be purged out of the gas-carrying system or from parts of the gas-carrying system using the other gas (purge gas), through the gas line (16) leading to the atmosphere.

2. Device according to Claim 1, **characterized by** a second line (16) being provided with a shut-off valve (12) and leading to the atmosphere.

## Revendications

1. Dispositif pour la distribution dosée d'un ou de plusieurs gaz à effet thérapeutique choisis dans le groupe constitué de NO, de CO, de mélanges de CO₂ destinés à la stimulation de la respiration, de mélanges de H2, de mélanges de N₂O, de mélanges de SF6, de nitroso-éthanol ou de gaz anesthésiants, comportant un premier conduit (L1) acheminant ledit gaz (gaz thérapeutique) vers un patient et un deuxième conduit (L2) dans lequel circule un autre gaz (gaz de balayage), **caractérisé par** un conduit (16) pourvu d'un robinet-vanne (14), ledit conduit menant vers l'extérieur et permettant ainsi d'évacuer, moyennant l'autre gaz (gaz de balayage), des substances nocives ou indésirables du système d'acheminement de gaz ou de parties du système d'acheminement de gaz par le biais du conduit de gaz (16) menant vers l'extérieur.

2. Dispositif selon la revendication 1, **caractérisé par** un deuxième conduit (16) menant vers l'extérieur et pourvu d'un robinet-vanne (12).
